# FASCICULE DE BREVET EUROPEEN

(11) **EP 1 011 626 B1**
(45) Date de publication et mention de la délivrance du brevet: **29.06.2005**
(21) Numéro de dépôt: 99909025.1
(22) Date de dépôt: 17.03.1999
(51) Int. Cl.: A61K 7/48, A61K 7/06

(54) **ymulsions cosmétiques solides eau-dans-huile contenant un tensioactif silicone du type alkyldiméthicone copolyol et une phase grasse comportant une huile de silicone et une cire de polyethylène**
Feste wasser-in-öl emulsionen enthaltend ein alkyldimethicone-copolyol tenside und eine ölphase die silikonöl und polyethylenwachs enthält
Solid water-in-oil cosmetic emulsion containing a silicone surfactant of alkyl-dimethicone copolyol type and an oily phase including at least one silicone oil and a polyethylene wax

(30) Priorité: 17.03.1998 FR 9803250
(43) Date de publication de la demande: 28.06.2000
(73) Titulaire: L'OREAL, 75008 Paris (FR)
(72) Inventeur: TERREN, Nadia, F-92340 Bourg-la-Reine (FR); ROUX, Marie-Martine, F-91200 Athis-Mons (FR); FAVRE, Sophie, F-94550 Chevilly-Larue (FR)
(74) Mandataire: Kromer, Christophe
(86) Numéro de dépôt international: PCT/FR1999/000609
(87) Numéro de publication internationale: WO 1999/047111

(56) Documents cités:
- EP-A- 0 374 332
- EP-A- 0 595 683
- D. G. KRZYSIK: "A New Silicone Emulsifier For Water-in-Oil Systems" DRUG COSMET. IND., vol. 146, no. 4, 1990, XP002090584
- F. BATTIONI: "Dalle emulsioni A/O alle emulsioni A/S e viceversa" IL PRODOTTO CHIMICO, vol. 28, no. 3, 1987, page 32-34 XP002090585
- CHEMICAL ABSTRACTS, vol. 116, no. 10, 9 mars 1992 (1992-03-09) Columbus, Ohio, US; abstract no. 91163, TAKADA, SUSUMU ET AL: "Solid-type water-in-oil emulsion cosmetics containing silicone oils, waxes, and spherical powders" XP002090586 cité dans la demande & JP 03 261707 A (SHISEIDO CO., LTD., JAPAN)

## Description

La présente invention concerne l'utilisation d'un tensio-actif siliconé du type alkyldiméthicone copolyol pour la préparation d'émulsions cosmétiques solides eau-dans-huile, ainsi que les émulsions cosmétiques solides eau-dans-huile, en particulier les fonds de teint.

Dans le domaine cosmétique, les émulsions eau-dans-huile (E/H) sont couramment utilisées car elles permettent de former à la surface de la peau un film qui prévient la perte d'eau transépidermique et protège la peau des agressions extérieures.

Compte tenu des exigences des consommateurs vis-à-vis de ce type d'émulsions, qui doivent avoir en même temps une bonne qualité cosmétique qui se traduit en terme d'aspect, de texture et de facilité d'application, de bonnes propriétés protectrices, une bonne tenue, ainsi qu'une bonne résistance à la sueur et au sébum, il est très avantageux de pouvoir obtenir des émulsions présentant toutes ces propriétés sans présenter les inconvénients des émulsions E/H classiques.

Les émulsions solides du type fond de teint comprennent généralement des corps gras tels que des huiles et des cires solides, de l'eau et une phase particulaire généralement composée de charges et de pigments.

Ces compositions, lorsqu'elles sont appliquées sur la peau, présentent toutefois l'inconvénient de transférer, c'est-à-dire de se déposer au moins en partie, en laissant une trace, sur certains supports avec lesquels elles peuvent être mises en contact, et notamment un vêtement ou la peau. Il s'ensuit une persistance médiocre du film sur la peau, d'où la nécessité de renouveler régulièrement l'application de la composition de fond de teint.

Un autre inconvénient des compositions de l'art antérieur réside dans une mauvaise dispersion des pigments, conduisant à une émulsion qui n'est pas homogène.

La demande de brevet JP-A-03261707 décrit des compositions cosmétiques solides du type émulsion eau-dans-huile contenant des huiles de silicone, des cires solides et de l'eau, et renfermant également des poudres sphériques.

Les émulsifiants utilisés peuvent être des organopoly-siloxanes modifiés par des polyoxyalcoylènes comme les diméthicone copolyols.

Les émulsions solides obtenues, à savoir des fonds de teint, sont, non homogènes, grossières, d'aspect microscopique et macroscopique non conformes et les pigments ne sont pas bien dispersés.

La présente invention a pour but de pallier ces inconvénients et propose une émulsion solide E/H homogène, dans laquelle les pigments, les colorants et les huiles sont bien dispersés, douce, glissante, ayant une très bonne tenue ainsi qu'une bonne persistance sur la peau.

La demanderesse a découvert de manière surprenante et inattendue qu'en utilisant un tensio-actif siliconé particulier du type alkyldiméthicone copolyol, en association avec au moins une huile de silicone et au moins une cire particulière, on pouvait obtenir une émulsion solide eau-dans-huile présentant les caractéristiques recherchées et qui présente également l'avantage de ne pas transférer.

Le document EP-A-59568 décrit une émulsion eau-dans-huile à phase continue fluorohydrocarbonée comprenant un tensioactif siliconé et éventuellement des huiles et des cires.

l'invention a donc pour objet est une émulsion cosmétique solide eau-dans-huile selon la revendication 1.

L'invention a également pour objet un procédé de maquillage de la peau et/ou du cuir chevelu, caractérisé par le fait qu'on applique sur la peau et/ou sur le cuir chevelu une émulsion solide telle que définie ci-dessus.

La demanderesse a constaté que l'émulsion solide selon l'invention s'applique et s'étale facilement de façon homogène, présente de bonnes propriétés d'hydratation et de bonnes propriétés cosmétiques, car elle est douce et glissante. Le film obtenu présente également une texture légère et reste confortable à porter tout au long de la journée.

De plus, l'émulsion appliquée sur la peau présente l'avantage de ne pas migrer dans les plis de la peau et/ou les rides du visage.

L'émulsion selon l'invention présente une texture homogène. On peut ajouter à l'émulsion selon l'invention des additifs tout en conservant une émulsion stable. L'émulsion selon l'invention est donc compatible avec un grand nombre d'adjuvants cosmétiques.

Le tensio-actif siliconé du type alkyldiméthicone copolyol utilisé selon l'invention a pour formule : 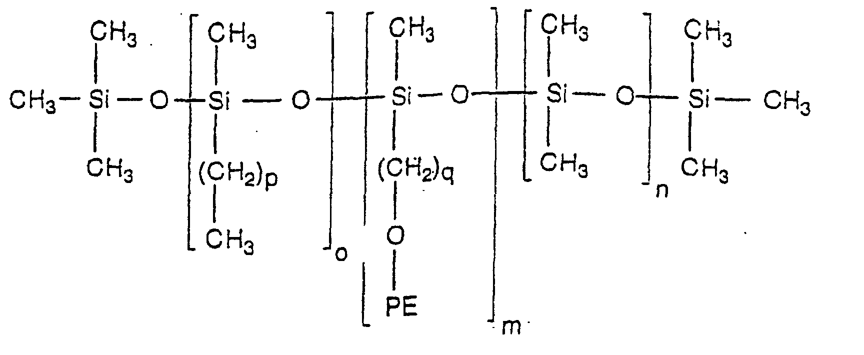 dans laquelle :
PE = (-C2H4Ox (-C3H6O)y - H,
x = 0 à 50,
y = 0 à 30, x et y n'étant pas simultanément 0,
o = 1 à 100,
m = 1 à 40,
n = 1 à 200,
p = 1 à 17 et
q = 1 à 5.
Et de préférence,
o = 1 à 25,
m = 1 à 10,
n = 1 à 100.

De tels tensio-actifs siliconés sont des produits commerciaux, et on peut citer à titre d'exemples les composés vendus sous les dénominations :
- "ABIL WE 09" par la Société GOLDSCHMIDT qui répond à la formule ci-dessus et pour lequel :
   o = 21,
   m = 4,
   n = 73,
- "ABIL WS 08" et "ABIL EM 90", par la Société GOLDSCHMIDT,
- "218-1138", par la Société GENERAL ELECTRIC, qui répond à la formule ci-dessus et pour lequel
   PE = (C₂H₄O)₁₂-H
   o = 2
   m = 8
   n = 20
   p = 9
   q = 3.

On utilise de préférence le produit "ABIL WE 09", qui est un mélange de cétyldiméthicone copolyol (dénomination CTFA), de polyglycéryl-4 isostéarate et de laurate d'hexyle (33,3 % / 33,3 % /33,4 %).

Le tensio-actif siliconé alkyldiméthicone copolyol est utilisé à raison de 0,5 à 40% en poids sur la base du poids total de l'émulsion, et de préférence à raison de 2 à 12% en poids.

L'émulsion selon l'invention comprend avantageusement 10 à 40 % en poids, et de préférence 18 à 30 % en poids, par rapport au poids total de l'émulsion, d'une huile.

L'émulsion selon l'invention comprend au moins une huile de silicone.

A titre d'exemples d'huiles de silicones utilisées dans l'invention, on peut citer :
- les silicones volatiles cycliques ayant de 3 à 8 atomes de silicium et de préférence 4 à 6, comme par exemple le cyclotétra-diméthylsiloxane, le cyclopentadiméthylsiloxane ou le cyclohexa-diméthylsiloxane;
- les cyclocopolymères du type diméthylsiloxane/méthyl-alkylsiloxane, tels que la "silicone FZ 3109", vendue par la Société UNION CARBIDE, qui est un cyclocopolymère diméthylsiloxane/méthyl -octylsiloxane;
- les silicones volatiles linéaires ayant de 2 à 9 atomes de silicium, par exemple l'hexaméthyldisiloxane, l'hexylheptaméthyl-trisiloxane et l'octylheptaméthyltrisiloxane;
- les polyalkylsiloxanes à groupements terminaux triméthyl-silyle, de préférence ceux dont la viscosité à 25°C est inférieure ou égale à 0,06 m²/s, parmi lesquels on peut citer les polydiméthylsiloxanes linéaires, et notamment ceux vendus sous la dénomination "DOW CORNING FLUID 200" par la Société DOW CORNING, et les alkylméthylpolysiloxanes tels que la cétyldiméthicone (dénomination CTFA) ;
- les huiles de silicone phénylées.

Les huiles de silicones volatiles sont préférées pour être utilisées dans l'invention.

A titre d'huiles volatiles utilisées préférentiellement selon l'invention, on peut également citer les huiles hydrocarbonées ayant de 8 à 16 atomes de carbone, et notamment les huiles isoparaffiniques volatiles en C8-C16 comme l'isododécane, l'isodécane et l'isohexadécane.

L'émulsion selon l'invention peut également contenir d'autres huiles et corps gras pâteux.

Les composés gras pâteux peuvent être définis à l'aide d'au moins l'une des propriétés physico-chimiques suivantes :
- une viscosité de 0,1 à 40 Pa.s, de préférence 0,5 à 25 Pa.s, mesurée à 40°C avec un viscosimètre rotatif CONTRAVES TV équipé d'un mobile MS-r3 ou MS-r4, à la fréquence de 60 Hz,
   - un point de fusion de 25-70°C, de préférence 25-55°C.

Parmi les autres huiles pouvant être utilisées selon l'invention, on peut citer :
- les huiles minérales telles que l'huile de paraffine, l'huile de vaseline,
- les huiles animales telles que le perhydrosqualène,
- les huiles végétales telles que les huiles d'abricot, de sésame, d'amande douce, de calophyllum, de palme, de ricin, d'avocat, de jojoba, d'olive ou de germes de céréales telles que l'huile de germes de blé,
- les esters ramifiés en C8-C16 comme le néopentanoate d'iso-hexyle,
- les esters et les éthers de synthèse comme les huiles de formule R1COOR2 dans laquelle R1 représente le reste d'un acide gras supérieur comportant de 6 à 29 atomes de carbone et R2 représente une chaîne hydrocarbonée contenant de 3 à 30 atomes de carbone, telles que l'huile de Purcellin, le myristate d'isopropyle, le palmitate d'éthyl-2 hexyle, le stéarate d'octyl-2 dodécyle, l'érucate d'octyl-2-dodécyle, l'isostéarate d'isostéaryle, le propionate d'arachidyle, le benzoate d'octyl-2 dodécyle; les esters hydroxylés comme l'isostéaryl lactate, l'octylhydroxystéarate, l'hydroxystéarate d'octyldodécyle, le diisostéarylmalate, le citrate de triisocétyle ; les esters de polyols comme le dioctanoate de propylène glycol, le diheptanoate de néopentylglycol, le diisononanoate de diéthylène glycol et les esters du pentaérythritol ;
- les alcools gras ayant de 12 à 16 atomes de carbone comme l'octyldodécanol, le 2-butyloctanol, le 2-hexyldécanol, le 2-undécylpentadécanol, l'alcool oléique ;
- les huiles fluorées parmi lesquelles on peut citer les perfluoropolyéthers comme les produits vendus sous la dénomination commerciale "FOMBLIN" par la société MONTEFLUOS, ainsi que les silicones fluorées telles que les trifluorométhyl (C1-C4) alkyl diméthicones, par exemple celle vendue sous la dénomination commerciale "X 22819" par la société SHIN ETSU ; et
- leurs mélanges.

L'émulsion cosmétique selon l'invention comprend avantageusement 3 à 15 % en poids, et de préférence 3 à 10 % en poids, sur la base du poids total de l'émulsion, d' une cire,

A titre de cires additionnelles pouvant être utilisées selon l'invention, on peut citer les cires d'origine animale telles que la cire d'abeilles, le spermaceti, la cire de lanoline et les dérivés de lanoline, les cires végétales telles que la cire de Carnauba, de Candellila, d'Ouricury, du Japon, le beurre de cacao ou les cires de fibres de liège ou de canne à sucre, les cires minérales, par exemple de paraffine, de vaseline, de lignite ou les cires microcristallines ou les ozokérites, les cires synthétiques parmi lesquelles les cires de polytétrafluoroéthylène et les cires obtenues par synthèse de Fisher-Tropsch ou encore les cires de silicone, les huiles hydrogénées concrètes à 25°C telles que l'huile de ricin hydrogénée, l'huile de jojoba hydrogénée, l'huile de palme hydrogénée, le suif hydrogéné, l'huile de coco hydrogénée et les esters gras concrets à 25°C comme le stéarate d'alkyle en C20-C40 vendu sous la dénomination commerciale "KESTER WAX K82H" par la société KOSTER KEUNEN.

Les cires de silicone utilisables dans la composition selon l'invention peuvent être des polysiloxanes linéaires substitués. On peut citer, par exemple, les cires de silicone polyéther, les alkyl ou alkoxy-diméthicones ayant de 16 à 45 atomes de carbone. On peut également citer les alkyl méthicones comme la C30-C45 alkyl méthicone vendue sous la dénomination commerciale "AMS C 30" par DOW CORNING.

De préférence, on utilise une cire ou un mélange de cires apte à conférer à l'émulsion solide selon l'invention une force de pénétration supérieure ou égale à 50 grammes (g).

Dans la présente demande, cette force de pénétration est mesurée selon le protocole suivant : en fin de préparation de l'émulsion, cette dernière est coulée dans une coupelle et est maintenue 24 heures à 20° C. On mesure alors sur cette émulsion solide la force de pénétration à l'aide d'un appareil analyseur de texture de marque STEVENS, au mobile de mesure TA24, diamètre de 4 mm, à une vitesse de pénétration de 0,5 mm/s et à la profondeur de pénétration présélectionnée de 2 mm. La force de pénétration exprimée en grammes se lit sur l'appareil.

L'émulsion selon l'invention comprend de la cire de polyéthylène. De préférence, l'émulsion comprend un mélange de cire de polyéthylène et de cire choisie parmi l'huile de jojoba hydrogénée, l'ozokérite ou leurs mélanges. Encore plus préférentiellement, l'émulsion selon l'invention comprend un mélange de cire de polyéthylène et d'huile de jojoba hydrogénée.

La phase aqueuse de l'émulsion selon l'invention peut représenter 0,5 à 60 % en poids du poids total de l'émulsion.

Elle peut comprendre de l'eau ou une eau florale telle que de l'eau de bleuet.

La phase aqueuse selon l'invention peut comprendre 0 à 14 % en poids de monoalcools inférieurs en C2-C6 et/ou de polyols tels que le glycérol, le butylèneglycol, l'isoprèneglycol et le propylèneglycol et des agents de stabilisation de l'émulsion, par exemple le chlorure de sodium, le dichlorure de magnésium et le sulfate de magnésium.

En outre, l'émulsion selon l'invention peut comprendre un ou plusieurs agents épaississants dans des concentrations allant de préférence de 0 à 6% en poids par rapport au poids total de l'émulsion.

L'émulsion selon l'invention peut également comprendre une phase particulaire qui peut comprendre des pigments et/ou des nacres et/ou des charges habituellement utilisés dans le domaine de la cosmétique et du maquillage. L'homme du métier veillera cependant à sélectionner ces composés pour un transfert minimum.

Les pigments peuvent être présents dans l'émulsion à raison de 0 à 30 % en poids, par rapport au poids total de l'émulsion, et de préférence à raison de 2 à 20 %. Ils peuvent être blancs ou colorés, minéraux et/ou organiques, de taille usuelle ou nanométrique. On peut citer, parmi les pigments et les nanopigments minéraux, les dioxydes de titane, de zirconium ou de cérium, ainsi que les oxydes de zinc, de fer ou de chrome, les nanotitanes et le bleu ferrique. Parmi les pigments organiques, on peut citer le noir de carbone, et les laques de baryum, strontium, calcium, aluminium et le carmin de cochenille.

Par charges, il faut comprendre des particules incolores ou blanches, minérales ou de synthèse, lamellaires ou non lamellaires. Par nacres, il faut comprendre des particules irisées, notamment produites par certains mollusques dans leur coquille ou bien synthétisées. Ces charges et nacres servent notamment à modifier la texture de la composition.

Les charges peuvent être présentes dans l'émulsion à raison de 0 à 25 % en poids, par rapport au poids total de l'émulsion, de préférence 0 à 10 %. On peut citer notamment le talc, le mica, la silice, le kaolin, le Téflon, l'amidon, le nitrure de bore, les poudres de Nylon (ORGASOL notamment) et de polyéthylène, les microsphères de copolymères telles que l'Expancel (NOBEL INDUSTRIE), le Polytrap (DOW CORNING) et les microbilles de résine de silicone (Tospearl de TOSHIBA, par exemple).

Les nacres peuvent être présentes dans l'émulsion à raison de 0 à 20 % en poids, par rapport au poids total de l'émulsion, de préférence de 2 à 15 %.

Les nacres utilisables selon l'invention sont par exemple le mica recouvert d'oxyde de titane, d'oxyde de fer, de pigment naturel ou d'oxychlorure de bismuth, ainsi que le mica titane coloré.

L'émulsion selon l'invention peut comprendre en outre tous additifs usuellement utilisés dans le domaine cosmétique, tels que des antioxydants, des colorants, des parfums, des huiles essentielles, des conservateurs, des actifs cosmétiques, des hydratants, des vitamines, des sphingolipides, des filtres solaires, des polymères liposolubles, notamment hydrocarbonés, tels que le polybutène, les polyalkylènes, les polyacrylates et les polymères siliconés compatibles avec les corps gras. Bien entendu, l'homme du métier veillera à choisir ce ou ces éventuels composés complémentaires, et/ou leur quantité, de manière telle que les propriétés avantageuses de la composition selon l'invention ne soient pas, ou substantiellement pas, altérées par l'adjonction envisagée. Ces additifs peuvent être présents dans la composition à raison de 0 à 15% en poids.

Les émulsions selon l'invention peuvent se présenter sous la forme d'un produit cosmétique et notamment sous la forme d'un produit de maquillage, en particulier un fond de teint, un fard à joues ou à paupières, ou un rouge à lèvres.

Elles peuvent également se présenter sous forme non colorée, contenant éventuellement des actifs cosmétiques.

Les exemples qui suivent servent à illustrer l'invention sans toutefois présenter un caractère limitatif. Dans ces exemples, les quantités sont données en pourcentage de poids par rapport au poids total de la composition.

### EXEMPLE 1 :

La Demanderesse a réalisé le fond de teint suivant:

### PHASE H

- Huile de jojoba hydrogénée 5,6 %
- Conservateur 0,3 %
- Cire de polyéthylène (PM : 500) 2,9 %
- Cire de polytétrafluoroéthylène 7 %
- Mélange de poly méthylcétyl diméthyl méthyl-siloxane oxyéthyléné oxypropyléné, isostéarate polyglycérolé (4 moles) et laurate d'hexyle, vendu sous la dénomination "ABIL WE 09" par la Société GOLDSCHMIDT 9 %

### SILICONE S

- Cyclohexadiméthylsiloxane (viscosité : 8.10-6 m2/s) 24,3 %

### PIGMENTS P

- Oxydes de fer 2,9 %
- Oxyde de titane 7,1 %

### PHASE E

- Eau déminéralisée stérilisée 38,5 %
- Propylèneglycol 1 %
- Conservateur 0,4 %
- Sulfate de magnésium, 7 H2O 1 %

On pèse ensemble les composés de la phase H que l'on chauffe à 100°C.
Après homogénéisation, on la refroidit à 80 °C puis on additionne la silicone S.
Les pigments P sont ensuite dispersés dans le mélange H+S.
Après homogénéisation, on ajoute lentement la phase E, préalablement chauffée à 80 °C, en agitant à l'aide d'un agitateur type Moritz et en conservant au cours de l'addition une température minimale de 75 °C.
On coule le produit et on obtient un fond de teint compact homogène, qui s'étale bien, qui conduit à un maquillage très naturel et très doux, qui présente une bonne tenue cosmétique et qui ne transfère pas.

Cette composition a une force de pénétration, mesurée sur un appareil STEVENS comme décrit dans le texte ci-dessus, supérieure à 50 grammes.

### EXEMPLE 2 : COMPARATIF: Fond de teint selon l'Art antérieur

On a reproduit l'exemple 5 de la demande JP-A-03261707.

### PHASE H

- Paraffine (cire minérale) 4 %
- Mélange diméthicone copolyol, cyclopentasiloxane, eau (10/88/2), vendu sous la dénomination "Q2-3225 C" par la Société DOW CORNING 30 %
- Camphre 0,1 %
- Menthol 0,1 %
- Cellulose microcristalline 3 %

### PIGMENTS P

- Dioxyde de titane enrobé de polydiméthylsiloxane 15 %
- Oxyde de fer jaune enrobé de polydiméthylsiloxane 3 %
- Oxyde de fer rouge enrobé de polydiméthylsiloxane 1 %
- Oxyde de fer noir enrobé de polydiméthylsiloxane 0,2 %

### PHASE E

- Eau 33 %
- Ethanol 5 %
- Polyéthylèneglycol 5 %
- p-hydroxybenzoate de méthyle 0,3 %

On prépare un fond de teint compact selon le mode opératoire ci-dessus. On obtient un fond de teint de couleur hétérogène, qui est inhomogène, dont les pigments sont mal dispersés et qui est rêche et sec à l'application.

### EXEMPLE 3 :

La Demanderesse a réalisé les deux compositions A, et B, suivantes, en faisant varier la nature de la cire :

### PHASE H

- cire 6,3 %
- conservateur 0,3 %
- Mélange de poly méthylcétyl diméthyl méthyl-siloxane oxyéthyléné oxypropyléné, isostéarate polyglycérolé (4 moles) et laurate d'hexyle, vendu sous la dénomination "ABIL WE 09" par la Société GOLDSCHMIDT 9 %

### SILICONE S

- Cyclohexadiméthylsiloxane (viscosité : 8.10-6 m2/s) 24,3 %

### PIGMENTS P

- Oxydes de fer 2,9 %
- Oxyde de titane 7,1 %

### PHASE E

- Eau déminéralisée stérilisée 38,5 %
- Propylèneglycol 1 %
- Conservateur 0,4 %
- Sulfate de magnésium, 7 H2O 1 %
avec:

| Composition | A | B |
|---|---|---|
| Nature de la cire | polyéthylène | carnauba |
| Force de pénétration (en g) | 101 | 11 |
| Aspect après coulage à chaud | solide | mou |
| Observation au microscope | émulsion fine régulière | petits cristaux |

Ces deux émulsions ont été préparées comme dans l'exemple 1. La force de pénétration a été mesurée sur un appareil STEVENS comme décrit dans le texte ci-dessus.

L'émulsion B (pas conforme à l'invention) qui ne comprend que de la cire de carnauba ne permet pas d'obtenir une composition suffisamment solide.

## Revendications

1. Emulsion cosmétique solide, du type eau-dans-huile, **caractérisée par le fait qu'**elle comporte une phase aqueuse émulsionnée à l'aide d'un tensio-actif siliconé de type alkyldiméthicone copolyol de formule: 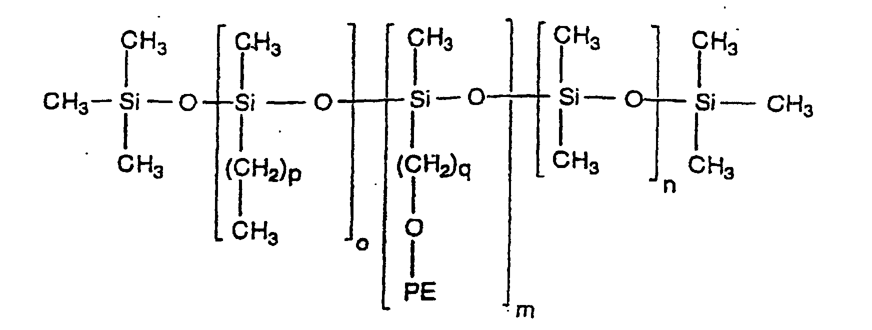 dans laquelle :
PE = (-C2H4O)x (-C3H6O)y - H,
x = 0 à 50,
y = 0 à 30, x et y n'étant pas simultanément 0,
o = 1 à 100,
m = 1 à 40,
n = 1 à 200,
p = 1 à 17 et
q = 1 à 5.
dans une phase grasse contenant au moins une huile de silicone et au moins une cire de polyéthyle

2. Emulsion selon la revendication 1, **caractérisée par le fait que** :
o = 1 à 25,
m = 1 à 10,
n=1à100.

3. Emulsion selon la revendication 2, **caractérisée par le fait que** :
o = 21,
m = 4,
n = 73,

4. Emulsion selon la revendication 3, **caractérisée par le fait que** le tensio-actif siliconé est un mélange de cétyldiméthicone copolyol, de polyglycéryl-4 isostéarate et de laurate d'hexyle.

5. Emulsion selon l'une quelconque des revendications 1 à 4, **caractérisée par le fait qu'**elle contient 0,5 à 40 % et de préférence 2 à 12 % en poids de tensio-actif siliconé alkyldiméthicone copolyol, sur la base du poids total de l'émulsion.

6. Emulsion selon la revendication 1, **caractérisée par le fait que** l'huile de silicone est choisie parmi les silicones volatiles cycliques ayant de 3 à 8 atomes de silicium, les silicones volatiles linéaires ayant de 2 à 9 atomes de silicium, les cyclocopolymères diméthylsiloxane/ méthylalkylsiloxane, les polyalkylsiloxanes à groupements terminaux triméthyl-silyle et les huiles de silicone phénylées.

7. Emulsion selon la revendication 1 ou 6, **caractérisée par le fait que** l'huile de silicone est une huile de silicone volatile.

8. Emulsion selon la revendication 7, **caractérisée par le fait que** l'huile de silicone volatile est choisie parmi le cyclotétradiméthyl-siloxane, le cyclopentadiméthylsiloxane, le cyclohexadiméthylsiloxane, l'hexaméthyldisiloxane, l'hexylhepta-méthyltrisiloxane et l'octylheptaméthyltrisiloxane.

9. Emulsion selon l'une quelconque des revendications 1 à 8, **caractérisée par le fait que** la phase grasse comprend une isoparaffine volatile.

10. Emulsion selon la revendication 9, **caractérisée par le fait que** l'isoparaffine volatile est une isoparaffine en C8-C16 choisie parmi l'isododécane, l'isodécane et l'isohexadécane.

11. Emulsion selon l'une quelconque des revendications 1 à 10, **caractérisée par le fait que** la phase grasse comprend en outre des huiles minérales, des huiles d'origine animale, des huiles végétales, des esters ramifiés en C8-C16, des esters et éthers de synthèse , des esters hydroxylés, des esters de polyols, des alcools gras, des huiles fluorées et leurs mélanges.

12. Emulsion selon l'une quelconque des revendications 1 à 11, **caractérisée par le fait qu'**elle contient 10 à 40 % et de préférence 18 à 30 % en poids d'huile(s), sur la base du poids total de l'émulsion.

13. Emulsion selon l'une quelconque des revendications 1 à 12, **caractérisée par le fait que** la cire est apte à conférer à l'émulsion une force de pénétration supérieure ou égale à 50 grammes.

14. Emulsion selon l'une quelconque des revendications 23 à 37, **caractérisée par le fait qu'**elle comprend un mélange de cire de polyéthylène et de cire choisie parmi l'huile de jojoba hydrogénée, l'ozokérite ou leurs mélanges.

15. Emulsion selon l'une quelconque des revendications précédentes, **caractérisée par le fait que** la cire est un mélange de cire de polyéthylène et d'huile de jojoba hydrogénée.

16. Emulsion selon l'une quelconque des revendications précédentes, **caractérisée par le fait qu'**elle contient 3 à 15 % et de préférence 3 à 10 % en poids de cire(s), sur la base du poids total de l'émulsion.

17. Emulsion selon l'une quelconque des revendications précédentes, **caractérisée par le fait que** la phase grasse comprend en outre des pigments et/ou des nacres et/ou des charges sélectionnés pour un transfert minimum.

18. Emulsion selon l'une quelconque des revendications précédentes **caractérisée par le fait que** la phase aqueuse représente 0,5 à 60 % du poids total de l'émulsion.

19. Emulsion selon l'une quelconque des revendications précédentes, **caractérisée par le fait que** la phase aqueuse comprend de l'eau ou une eau florale, 0 à 14 % en poids par rapport au poids total de la phase aqueuse, de monoalcools inférieurs en C2-C6 et/ou de polyols et 0 à 6 % en poids par rapport au poids total de l'émulsion d'un agent épaississant, ainsi qu'éventuellement des agents de stabilisation de l'émulsion.

20. Emulsion selon l'une quelconque des revendications précédentes, **caractérisée par le fait qu'**elle comprend en outre au moins un additif choisi parmi les antioxydants, les colorants, les parfums, les huiles essentielles, les conservateurs, les actifs cosmétiques, les hydratants, les vitamines, les sphingolipides, les filtres solaires et les polymères liposolubles.

21. Emulsion selon l'une quelconque des revendications précédentes, **caractérisée par le fait qu'**elle se présente sous le format d'un produit de maquillage, et notamment un fond de teint, un fard à joues ou à paupières, un rouge à lèvres.

22. Procédé de maquillage de la peau et/ou du cuir chevelu, **caractérisé par le fait qu'**il consiste à appliquer sur la peau et/ou le cuir chevelu une émulsion solide telle que définie dans l'une quelconque des revendications précédentes.

## Claims

1. Solid cosmetic emulsion of the water-in-oil type, **characterized in that** it comprises an aqueous phase emulsified using a silicone surfactant of alkyl dimethicone copolyol type of formula: 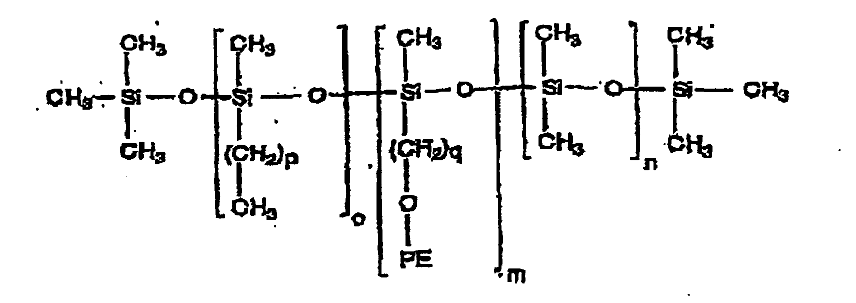 in which:
PE = (-C₂H₄O)ₓ(-C₃H₆O)_{y}-H,
x = 0 to 50,
y = 0 to 30, x and y not simultaneously being 0,
o = 1 to 100,
m = 1 to 40,
n = 1 to 200,
p = 1 to 17 and
q = 1 to 5,
in a fatty phase comprising at least one silicone oil and at least one polyethylene wax.

2. Emulsion according to Claim 1,
**characterized in that**:
o = 1 to 25,
m = 1 to 10,
n = 1 to 100.

3. Emulsion according to Claim 2,
**characterized in that**:
o = 21,
m = 4,
n = 73.

4. Emulsion according to Claim 3,
**characterized in that** the silicone surfactant is a mixture of cetyl dimethicone copolyol, of polyglyceryl-4 isostearate and of hexyl laurate.

5. Emulsion according to any one of Claims 1 to 4, **characterized in that** it comprises 0.5 to 40% and preferably 2 to 12% by weight of alkyl dimethicone copolyol silicone surfactant, on the basis of the total weight of the emulsion.

6. Emulsion according to Claim 1,
**characterized in that** the silicone oil is chosen from volatile cyclic silicones having from 3 to 8 silicon atoms, volatile linear silicones having from 2 to 9 silicon atoms, dimethylsiloxane/methylalkylsiloxane cyclocopolymers, polyalkylsiloxanes with trimethylsilyl end groups and phenylated silicone oils.

7. Emulsion according to Claim 1 or 6,
**characterized in that** the silicone oil is a volatile silicone oil.

8. Emulsion according to Claim 7,
**characterized in that** the volatile silicone oil is chosen from cyclotetradimethylsiloxane, cyclopentadimethylsiloxane, cyclohexadimethylsiloxane, hexamethyldisiloxane, hexylheptamethyltrisiloxane and octylheptamethyltrisiloxane.

9. Emulsion according to any one of Claims 1 to 8, **characterized in that** the fatty phase comprises a volatile isoparaffin.

10. Emulsion according to Claim 9,
**characterized in that** the volatile isoparaffin is a C₈-C₁₆ isoparaffin chosen from isododecane, isodecane and isohexadecane.

11. Emulsion according to any one of Claims 1 to 10, **characterized in that** the fatty phase additionally comprises mineral oils, oils of animal origin, vegetable oils, branched C₈-C₁₆ esters, synthetic esters and ethers, hydroxylated esters, polyol esters, fatty alcohols, fluorinated oils and their mixtures.

12. Emulsion according to any one of Claims 1 to 11, **characterized in that** it comprises 10 to 40% and preferably 18 to 30% by weight of oil(s) on the basis of the total weight of the emulsion.

13. Emulsion according to any one of Claims 1 to 12, **characterized in that** the wax is capable of conferring, on the emulsion, a penetration force of greater than or equal to 50 grams.

14. Emulsion according to any one of Claims 1 to 13, **characterized in that** it comprises a mixture of polyethylene wax and of wax chosen from hydrogenated jojoba oil, ozokerite or their mixtures.

15. Emulsion according to any one of the preceding claims, **characterized in that** the wax is a mixture of polyethylene wax and of hydrogenated jojoba oil.

16. Emulsion according to any one of the preceding claims, **characterized in that** it comprises 3 to 15% and preferably 3 to 10% by weight of wax(es) on the basis of the total weight of the emulsion.

17. Emulsion according to any one of the preceding claims, **characterized in that** the fatty phase additionally comprises pigments and/or pearlescent agents and/or fillers selected for minimum transfer.

18. Emulsion according to any one of the preceding claims, **characterized in that** the aqueous phase represents 0.5 to 60% of the total weight of the emulsion.

19. Emulsion according to any one of the preceding claims, **characterized in that** the aqueous phase comprises water or a floral water, 0 to 14% by weight, with respect to the total weight of the aqueous phase, of lower C₂-C₆ monoalcohols and/or of polyols and 0 to 6% by weight, with respect to the total weight of the emulsion, of a thickening agent, as well as, optionally, agents for the stabilization of the emulsion.

20. Emulsion according to any one of the preceding claims, **characterized in that** it additionally comprises at least one additive chosen from antioxidants, colorants, fragrances, essential oils, preservatives, cosmetic active principles, moisturizers, vitamins, sphingolipids, sunscreen agents and fat-soluble polymers.

21. Emulsion according to any one of the preceding claims, **characterized in that** it is provided in the form of a make-up product and in particular a foundation, a blusher, an eyeshadow or a lipstick.

22. Process for making up the skin and/or the scalp, **characterized in that** it consists in applying, to the skin and/or the scalp, a solid emulsion as defined in any one of the preceding claims.

## Patentansprüche

1. Feste kosmetische Emulsion vom Wasser-in-Öl-Typ, **dadurch gekennzeichnet, dass** sie in einer Fettphase, die mindestens ein Siliconöl und mindestens ein Polyethylenwachs enthält, eine wässrige Phase aufweist, die mit einem grenzflächenaktiven Silicon vom Typ Alkyldimethiconcopolyol der folgenden Formel emulgiert ist: 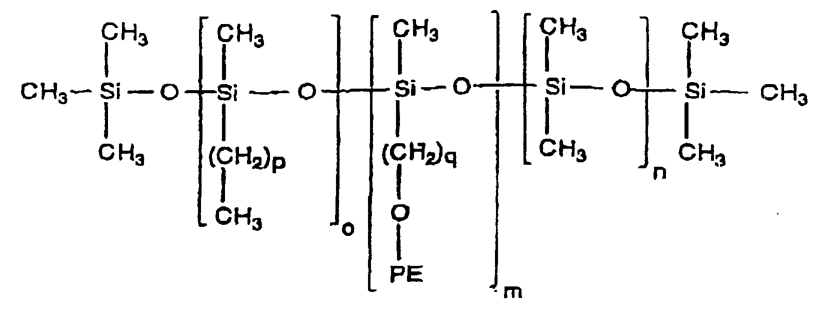 worin bedeuten:
PE = (-C₂H₄O)ₓ (-C₃H₆O)_{y} -H,
x = 0 bis 50,
y = 0 bis 30, wobei x und y nicht gleichzeitig 0 bedeuten,
o = 1 bis 100,
m = 1 bis 40,
n = 1 bis 200,
p = 1 bis 17 und
q = 1 bis 5.

2. Emulsion nach Anspruch 1, **dadurch gekennzeichnet, dass**:
o = 1 bis 25,
m = 1 bis 10,
n = 1 bis 100.

3. Emulsion nach Anspruch 2, **dadurch gekennzeichnet, dass**:
o = 21,
m = 4,
n = 73.

4. Emulsion nach Anspruch 3, **dadurch gekennzeichnet, dass** es sich bei dem grenzflächenaktiven Silicon um ein Gemisch von Cetyldimethiconcopolyol, Tetraglycerylisostearat und Hexyllaurat handelt.

5. Emulsion nach einem der Ansprüche 1 bis 4, **dadurch gekennzeichnet, dass** sie 0,5 bis 40 Gew.-% und vorzugsweise 2 bis 12 Gew.-% Alkyldimethiconcopolyol (grenzflächenaktives Silicon) auf der Basis des Gesamtgewichts der Emulsion enthält.

6. Emulsion nach Anspruch 1, **dadurch gekennzeichnet, dass** das Siliconöl unter den cyclischen flüchtigen Siliconen mit 3 bis 8 Siliciumatomen, geradkettigen flüchtigen Siliconen mit 2 bis 9 Siliciumatomen, Dimethylsiloxan/Methylalkylsiloxan-Cyclocopolymeren, Polyalkylsiloxanen mit endständigen Trimethylsilylgruppen und phenylierten Siliconölen ausgewählt ist.

7. Emulsion nach Anspruch 1 oder 6, **dadurch gekennzeichnet, dass** das Siliconöl ein flüchtiges Siliconöl ist.

8. Emulsion nach Anspruch 7, **dadurch gekennzeichnet, dass** das flüchtige Siliconöl unter Cyclotetradimethylsiloxan, Cyclopentadimethylsiloxan, Cyclohexadimethylsiloxan, Hexamethyldisiloxan, Hexylheptamethyltrisiloxan und Octylheptamethyltrisiloxan ausgewählt ist.

9. Emulsion nach einem der Ansprüche 1 bis 8, **dadurch gekennzeichnet, dass** die Fettphase ein flüchtiges Isoparaffin enthält.

10. Emulsion nach Anspruch 9, **dadurch gekennzeichnet, dass** das flüchtige Isoparaffin ein C₈₋₁₆-Isoparaffin ist, das unter Isododecan, Isodecan und Isohexadecan ausgewählt ist.

11. Emulsion nach einem der Ansprüche 1 bis 10, **dadurch gekennzeichnet, dass** die Fettphase ferner Mineralöle, Öle tierischer Herkunft, pflanzliche Öle, verzweigte C₈₋₁₆-Ester, synthetische Ester und Ether, hydroxylierte Ester, Polyolester, Fettalkohole, fluorierte Öle und deren Gemische enthält.

12. Emulsion nach einem der Ansprüche 1 bis 11, **dadurch gekennzeichnet, dass** sie 10 bis 40 % und vorzugsweise 18 bis 30 Gew.- % Öl(e) auf der Basis des Gesamtgewichts der Emulsion enthält.

13. Emulsion nach einem der Ansprüche 1 bis 12, **dadurch gekennzeichnet, dass** das Wachs geeignet ist, der Emulsion eine Eindringkraft von 50 g oder darüber zu geben.

14. Emulsion nach einem der Ansprüche 1 bis 13, **dadurch gekennzeichnet, dass** sie ein Gemisch aus Polyethylenwachs und einem Wachs enthält, das unter hydriertem Jojobaöl, Ozokerit oder deren Gemischen ausgewählt ist.

15. Emulsion nach einem der vorhergehenden Ansprüche, **dadurch gekennzeichnet, dass** das Wachs ein Gemisch aus Polyethylenwachs und hydriertem Jojobaöl ist.

16. Emulsion nach einem der vorhergehenden Ansprüche, **dadurch gekennzeichnet, dass** sie 3 bis 15 Gew.-% und vorzugsweise 3 bis 10 Gew.-% Wachs(e) auf der Basis des Gesamtgewichts der Emulsion enthält.

17. Emulsion nach einem der vorhergehenden Ansprüche, **dadurch gekennzeichnet, dass** die Fettphase ferner Pigmente und/oder Perlglanzpigmente und/oder Füllstoffe enthält, die im Hinblick auf einen möglichst geringen Transfer ausgewählt sind.

18. Emulsion nach einem der vorhergehenden Ansprüche, **dadurch gekennzeichnet, dass** die wässrige Phase 0,5 bis 60 % des Gesamtgewichts der Emulsion ausmacht.

19. Emulsion nach einem der vorhergehenden Ansprüche, **dadurch gekennzeichnet, dass** die wässrige Phase Wasser oder ein Blütenwasser, 0 bis 14 Gew.-% niedere C₂₋₆-Monoalkohole und/oder Polyole, bezogen auf das Gesamtgewicht der wässrigen Phase, und 0 bis 6 Gew.-% Verdickungsmittel, bezogen auf das Gesamtgewicht der Emulsion, sowie gegebenenfalls Stabilisatoren für die Emulsion enthält.

20. Emulsion nach einem der vorhergehenden Ansprüche, **dadurch gekennzeichnet, dass** sie ferner mindestens einen Zusatzstoff enthält, der unter den Antioxidantien, Farbmitteln, Parfums, etherischen Ölen, Konservierungsmitteln, kosmetischen Wirkstoffen, Hydratisierungsmitteln, Vitaminen, Sphingolipiden, Sonnenschutzfiltern und fettlöslichen Polymeren ausgewählt ist.

21. Emulsion nach einem der vorhergehenden Ansprüche, **dadurch gekennzeichnet, dass** sie als Produkt zum Schminken vorliegt, insbesondere als Make-up, Wangenrouge, Lidschatten oder Lippenstift.

22. Verfahren zum Schminken der Haut und/oder der Kopfhaut, **dadurch gekennzeichnet, dass** es darin besteht, auf die Haut und/oder die Kopfhaut eine feste Emulsion nach einem der vorhergehenden Ansprüche aufzutragen.
